# EUROPÄISCHE PATENTSCHRIFT

(11) **EP 2 128 128 B1**
(45) Veröffentlichungstag und Bekanntmachung des Hinweises auf die Patenterteilung: **29.07.2015**
(21) Anmeldenummer: 09006704.2
(22) Anmeldetag: 19.05.2009
(51) Int. Cl.: C07C 263/20, C07C 263/10, C07C 265/00

(54) **Ammoniumchloridabtrennung aus der Gasphase**
Separating ammonium chloride from the gas phase
Séparation du chlorure d'ammonium d'une phase gazeuse

(30) Priorität: 31.05.2008 EP 08009992
(43) Veröffentlichungstag der Anmeldung: 02.12.2009
(73) Patentinhaber: Bayer Intellectual Property GmbH, 40789 Monheim (DE)
(72) Erfinder: Hansen, Sven, Michael, Dr., 51373 Leverkusen (DE); Schneider, Ralf, Dr., 51375 Leverkusen (DE); Schwethelm, Bernd, 51381 Leverkusen (DE); Schiefer, Rolf, 51373 Leverkusen (DE); Dreher, Jürgen, 51061 Köln (DE)
(74) Vertreter: BIP Patents

(56) Entgegenhaltungen:
- EP-A- 1 403 248
- EP-A- 1 754 698
- DD-A1- 270 661

## Beschreibung

Die vorliegende Erfindung betrifft ein neues Verfahren zur Herstellung von Isocyanaten in der Gasphase, wobei die Standzeiten der Anlage durch selektive Abtrennung von Nebenprodukten wie Ammoniumchlorid durch Desublimation aus der Gasphase erreicht werden können.

Bei der Herstellung von Isocyanaten durch Phosgenierung der entsprechenden Amine in der Gasphase kommt es durch Eduktverunreinigungen bzw. Spaltreaktionen bei der Er- und Überhitzung der Amine beim Übergang in die Gasphase zur Bildung von Ammoniak, der mit bei der Isocyanatbildung freigesetztem Chlorwasserstoff zu Ammoniumchlorid reagieren kann.

Dies führt insbesondere in den Anlagenteilen der Gasaufbereitung zu Feststoffablagerungen und Druckverlusten, so dass die Anlage regelmäßig abgestellt und gereinigt werden muss.

Aus dem Stand der Technik ist dieses grundsätzliche Problem bei der Herstellung von Isocyanaten durch Gasphasenphosgenierung bekannt, Lösungen zur Beseitigung wurden allerdings noch nicht vorgeschlagen.

Grundsätzlich jedoch sind Maßnahmen zur Partikelabscheidung aus Rauchgasen bzw. Abluftströmen bekannt. Aufgrund des vorherrschenden physikalischen Abscheideprinzips werden übliche Staubabscheider in vier Grundtypen eingeteilt, wobei auch Mischformen angewendet werden (z. B. Naßelektrofilter).

In Massenkraftabscheidern (Zyklon, Absetz- u. Drehströmungskammer) werden die Massenkräfte Schwerkraft, Zentrifugalkraft u. Trägheitskraft genutzt. Sie werden in der Regel zur Produktrückführung od. als Vorabscheider eingesetzt. Das Prinzip der Naßabscheider (Venturi-Wäscher, Rotationswäscher, Strahlwäscher) beruht auf einer Anlagerung der Staubpartikel an dispergierte Flüssigkeitstropfen, deren Durchmesser mind. eine Größenordnung über dem der Staubpartikel liegt. Je nach Anwendung können die Wäscher gleichzeitig als Staubabscheider, Quenche, Befeuchter u. Absorber wirksam werden (z. B. Rohgasvorbehandlung zur Lösungsmittel-Abscheidung, Müllverbrennungsanlagen). Nachteilig ist eine häufig notwendige Nachbehandlung der dabei anfallenden Abwässer. Elektrofilter sind für Prozesse mit hoher Abgastemperatur und großen Abgasvolumenströmen, wie sie bei Großfeuerungsanlagen, Zement-Öfen und Eisenerz-Sinteröfen auftreten, die am häufigsten eingesetzten Anlagen. Die Staubpartikel werden in einem elektrischen Feld aufgeladen und wandern zur Niederschlagselektrode, wo sie abgeschieden werden. Filternde Abscheider nutzen die Filterwirkung von Geweben, Filzen, mineralischen Fasern oder Edelstahlfasern. Nachteilig insbesondere an den Separatoren, welche nach dem Prinzip eines Gaswäschers funktionieren, ist der vergleichsweise hohe Druckverlust.

EP 1754698 offenbart eine Gasphasenphosgenierung, bei der die Bildung von Nebenprodukten bereits in der Phase der Eduktvorbereitung verringert wird. Zu diesem Zweck wenden die zu phosgenierenden Amine vor der Phosgenierungsreaktion mittels eines Wärmetauschers verdampft. Der verwendete Wärmetauscher zeichnet sich durch eine speziell dimensionierte volumenspezifische Wärmetauscherfläche und einen speziell dimensionierten hydraulischen Durchmesser aus, die gemeinsam zu einer geringeren Verweilzeit der Amine im Tauscher und somit zu einer verringerten Zersetzung der Amine unter Ammoniak-Freisetzung führen. Damit geht eine verringerte Nebenproduktbildung im Verlauf der späteren Phosgenierungsreaktion einher.

DD 270661 A1 beschreibt die Desublimation von Stoffen aus Abgasegemischen mittels Wärmetauscher. Letztere umfassen drei Kondensationszonen mit steigender spezifischer Abscheidungsoberfläche, beginnend mit einer Zone mit Plattenwärmetauschern und endend mit einer Zone aus einer temperierbaren Aktivkohleschicht.

Überraschenderweise wurde nun gefunden, dass sich zur Abscheidung von Nebenprodukten wie Ammoniumhalogeniden aus Gasströmen, wie sie bei der Phosgenierung von Aminen in der Gasphase einschließlich der angeschlossenen Abtrennungs- und Aufarbeitungsschritte vorliegen, gerade das Prinzip der Re- oder Desublimation sehr erfolgreich einsetzen lässt, wodurch eine nahezu vollständige und selektive Abtrennung aus dem Gasraum erzielt wird.

Gegenstand der Erfindung ist daher ein Verfahren zur Herstellung von Isocyanaten durch Phosgenierung von Aminen in der Gasphase, bei dem Nebenprodukte durch Desublimation als Feststoff aus einem oder mehreren Gasströmen abgetrennt werden, worin das zu phosgenierende Amin und das Phosgen dem Reaktor zugeführt werden, das sich bildende isocyanathaltige Reaktionsgemisch unter Zusatz eines Quenchemittels dem Isocyanatwäscher zugeführt wird und nach Abtrennung des Isocyanats weiter über den Kondensator im Phosgenwäscher in Abgas und Phosgenstrom aufgetrennt wird, dadurch gekennzeichnet, dass die Desublimation im sich an die Reaktionszone anschließenden Gasweg in einem oder mehreren Plattenwärmetauschern durchgeführt wird, wobei der bzw. die Wärmetauscher so ausgelegt und das Verfahren in der Weise betrieben wird, dass die spezifische Gasgeschwindigkeit 2 m/s oder weniger bei Durchgang durch den oder die zur Desublimation eingesetzten Wärmetauscher beträgt und die Wärmetauscher mit einem Wärmeträgermedium betrieben werden, das eine Temperatur von 100°C bis - 40°C aufweist.

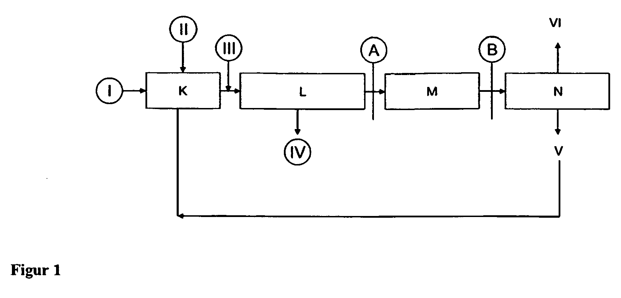

Figur 1 zeigt schematisch den Ablauf der erfindungsgemäßen Gasphasenphosgenierung, wobei mit (A) und (B) beispielhaft Stellen im Verfahren gekennzeichnet sind, an denen eine solche erfindungsgemäße Desublimation durchgeführt werden kann.

Das zu phosgenierende Amin (I) und das Phosgen (II) werden dem Reaktor (K) zugeführt. Das sich bildende isocyanathaltige Reaktionsgemisch wird unter Zusatz eines Quenchemittels (III), z.B. Monochlorbenzol dem Isocyanatwäscher (L) zugeführt und nach Abtrennung des Isocyanats (IV) weiter über den Kondensator (M) im Phosgenwäscher (N) in Abgas (V) und Phosgenstrom (VI) aufgetrennt.

Bevorzugte Nebenprodukte sind Ammoniumhalogenide, besonders bevorzugt ist Ammoniumchlorid.

Als Desublimieren bezeichnet man in der Thermodynamik das unmittelbare Übergehen eines Stoffes vom gasförmigen in den festen Aggregatzustand. Den Vorgang selbst bezeichnet man als Resublimation, Desublimation, Solidifikation oder auch Deposition.

Bevorzugtes Phosgen besitzt noch Restanteile an Kohlenmonoxid sowie ferner einen Gehalt an Chlorwasserstoff von nicht mehr als 15 Gew.-%, bevorzugt 0,1 bis 10 Gew.-%, besonders bevorzugt 2 bis 8 Gew.-%.

Bevorzugt wird aus der Phosgenabtrennung rückgeführtes Phosgen nach Anreicherung mit Frisch-Phosgen aus der Phosgenerzeugung der Aminphosgenierung zugeführt.

Als Amine können im erfindungsgemäßen Verfahren sämtliche aminofunktionelle Verbindungen mit wenigstens einer, bevorzugt 1 bis 3 primären Aminogruppen eingesetzt werden, sofern sie in die Dampfform überführt werden können. Es ist dabei unerheblich ob die Amine aliphatischer, cycloaliphatischer, araliphatischer oder aromatischer Natur sind.

Vorgenannte aminofunktionelle Verbindungen weisen üblicherweise bis zu 18 Kohlenstoffatome auf, wobei, falls mehrere Aminogruppen im Molekül vorliegen, diese durch wenigstens 2 Kohlenstoffatome von einander getrennt sind.

Besonders geeignet sind zu diesem Zweck Diamine auf Basis von aliphatischen oder cycloaliphatischen Kohlenwasserstoffen mit 2 bis 18 Kohlenstoffatomen.

Beispiele hierfür sind 1,4-Diaminobutan, 1,6-Diaminohexan, 1,8-Diaminooctan, 1,10-Diaminodecan, 1,6-Diamino-3,3,5-trimethyl-hexan, 1-Amino-3,3,5-trimethyl-5-aminomethylcyclohexan (IPDA), 2,4-, oder 2,6-Diamino-1-methylcyclohexan und 4,4'-Diaminodicyclohexylmethan. Besonders bevorzugt sind 1,6-Diisocyanatohexan, 1-Isocyanato-3,3,5-trimethyl-5-(isocyanatomethyl)cyclohexan, und 4,4'-Di-(isocyanatocyclohexyl)-methan.

Ebenfalls als Ausgangsmaterialien geeignet sind beliebige (cyclo)aliphatische Triamine mit bis zu 22 Kohlenstoffatomen, soweit sie unter den Temperaturbedingungen des erfindungsgemäßen Verfahrens stabil sind und in die Dampfform überführt werden können. Beispielsweise können dies Triaminocyclohexan, Tris(aminomethyl)-cyclohexan, Triamino-methylcyclohexan sein. Ebenfalls geeignet sind 1,8-Diamino-4-(aminomethyl)octan, 1,6,11-Undecantriamin, 1,7-Diamino-4-(3-aminopropyl)heptan, 1,6-Diamino-3-(aminomethyl)-hexan oder 1,3,5-Tris(aminomethyl)-cyclohexan.

Ebenfalls können für das erfindungsgemäße Verfahren aromatische Amine verwendet werden, die bevorzugt ohne Zersetzung in die Gasphase überführt werden können. Beispiele für bevorzugte aromatische Amine sind Toluylendiamin (TDA), als 2,4- oder 2,6-Isomer oder als Gemisch davon, Diaminobenzol, 2,6-Xylidin, Napthylendiamin (NDA) und 2,4'- oder 4,4'-Methylen(di-phenylamin) (MDA) oder Isomerengemische davon. Bevorzugt sind 2,4- und/oder 2,6-TDA.

Bevorzugt werden Amine der vorgenannten Art eingesetzt, die unter den gewählten Prozessbedingungen weitestgehend ohne Zersetzung in die Gasphase überführt werden können. "weitestgehend ohne Zersetzung" meint in diesem Zusammenhang, dass höchstens 1 Gew.-%, bevorzugt höchstens 0,1 Gew.-%, besonders bevorzugt höchstens 0,05 Gew.-% des in die Verdampfung eingesetzten Amins während der Er- und Überhitzung unter Nebenproduktbildung wie Ammoniakabspaltung, Bildung sekundärer oder tertiärer Amine abreagiert.

Die Edukte Amin und Phosgen können jeweils auch zusammen mit einem Inertmedium in den Reaktionsraum eindosiert werden. Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im Allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Dichlorbenzol, Xylol oder Kohlenstoffdioxid, verwendet werden. Bevorzugt wird Stickstoff und/oder Chlorbenzol als Inertmedium verwendet.

Im Allgemeinen wird das Inertmedium in einer Menge eingesetzt, so dass das Verhältnis von Gasvolumen des Inertmedium zu Gasvolumen des Amins bzw. Phosgens bei 0,001 bis 5, bevorzugt 0,01 bis 3, besonders bevorzugt 0,1 bis 1 liegt. Bevorzugt wird das Inertmedium zusammen mit den Aminen in den Reaktionsraum eingeführt.

Das erfindungsgemäße Verfahren wird bevorzugt so durchgeführt, dass die Edukte Amin und Phosgen als auch das in der Reaktionszone entstehende Isocyanat unter den Reaktionsbedingungen in gasförmigem Zustand sind, das heißt, dass eine Bildung von Flüssigkeitströpfen bevorzugt ausgeschlossen ist.

Zur Bereitstellung der vorgenannten Reaktionsbedingungen betragen die Temperaturen in der Reaktionszone bevorzugt mehr als 200°C, besonders bevorzugt mehr als 260°C, ganz besonders mehr als 280°C. Die Temperaturobergrenze liegt dabei bevorzugt unterhalb 570°C, besonders bevorzugt unterhalb von 500°C.

Die Umsetzung von Phosgen mit Amin in der jeweiligen Reaktionszone erfolgt bei Absolutdrücken von mehr als 0,1 bar bis weniger als 20 bar, bevorzugt 0,5 bar bis 10 bar, besonders bevorzugt 0,7 bar bis 5 bar, ganz besonders bevorzugt 0,8 bis 3 bar.

Im allgemeinen ist der Druck in den Zuleitungen in die Reaktionszone höher, als der vorstehend angegebene Druck in der Reaktionszone selber. Bevorzugt ist der Druck in den Zuleitungen um 20 bis 2000 mbar, besonders bevorzugt von 30 bis 1000 mbar höher als in der Reaktionszone selbst.

Im allgemeinen ist der Druck in den sich an die eigentliche Reaktionszone anschließenden Bereichen des Verfahrens niedriger als in den Reaktionszonen selbst. Bevorzugt ist der Druck dort um 10 bis 500 mbar, besonders bevorzugt 30 bis 150 mbar, niedriger als in der Reaktionszone.

Bevorzugt werden die Edukte mit einer Strömungsgeschwindigkeit von je 3 bis 100 m/s, bevorzugt von 10 bis 50 m/s in und durch die Reaktionszone geleitet.

Die Strömungsgeschwindigkeiten der beiden Edukte werden innerhalb der vorgenannten Bereiche bevorzugt so eingestellt, dass in der Reaktionszone eine mittlere Kontaktzeit des Umsetzungsgemisches aus Aminen und Phosgen im allgemeinen von 0,01 Sekunden bis weniger als 15 Sekunden, bevorzugt von mehr als 0,04 Sekunden bis weniger als 10 Sekunden, besonders bevorzugt von mehr als 0,08 Sekunden bis weniger als 5 Sekunden erreicht wird. Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 250 charakterisiert.

Vorteilhafterweise werden die Abmessungen des Reaktionsraums und die Strömungsgeschwindigkeiten dabei so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Durch die turbulente Strömung wird eine enge Verweilzeit mit geringer Standardabweichung von unterhalb 10 %, bevorzugt unterhalb 6 % erreicht.

Bevorzugt besitzt die Reaktionszone keine beweglichen Einbauten.

Die Reaktionszone kann über seine Außenfläche temperiert werden. Um Produktionsanlagen mit hoher Anlagenkapazität zu bauen, können mehrere Reaktorrohre parallel geschaltet werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen. Bevorzugt findet die Umsetzung adiabat statt.

Nachdem das Reaktionsgemisch in der Reaktionszone umgesetzt wurde, ist eine rasche Abkühlung der Reaktionsgase nach der Phosgenierungsreaktion auf Temperaturen unter 150°C notwendig, um die Bildung unerwünschter Nebenprodukte durch den thermischen Zerfall von Di-/Triisocyanat oder durch eine Weiterreaktion durch Polymerisation zu vermeiden, da die gebildeten Di/Triisocyanate bei den Reaktionstemperaturen von 300 bis 570°C thermisch nicht stabil sind. Die Abkühlung auf Temperaturen von 100 bis 150 °C erfolgt in einer ein- oder mehrstufigen Wäsche (Quenche mit Waschkolonne) mit einem inerten Lösungsmittel, wie in EP-A1 1403248, Sp. 2, Z. 39 - Sp. 3, Z. 18 beschrieben .

Als Lösungsmittel sind bevorzugt Kohlenwasserstoffe, die gegebenenfalls mit Halogenatomen substituiert sind, geeignet, wie beispielsweise Chlorbenzol, Dichlorbenzol, und Toluol. Als Lösungsmittel wird besonders bevorzugt Monochlorbenzol eingesetzt. Als Lösungsmittel kann auch das Isocyanat oder eine Lösung des hergestellten Isocyanates, die auch über einen Wärmetauscher zur Energieabfuhr im Kreis gefahren werden kann, eingesetzt werden.

Bei der Wäsche wird das Isocyanat selektiv in die Waschlösung übergeführt. Aus dem verbleibenden isocyanatfreien Gas (überschüssiges Phosgen, Chlorwasserstoff, gegebenenfalls das Inertmedium und Lösungsmittel aus der Wäsche) wird das Lösungsmittel durch partielle Kondensation und anschließend das Phosgen z.B. mittels Absorption in Monochlorbenzol isoliert, zurückgewonnen und wieder dem Phosgen-Eduktstrom zugeführt. Der Chlorwasserstoff wird nach Reinigung gemäß Stand der Technik als Rohstoff weiterverwendet.

Die in der Quenche und Waschkolonne erhaltene konzentrierte Isocyanatlösung wird bevorzugt mittels Rektifikation von physikalisch (gelöstem) und chemisch gebundenem Chlorwasserstoff und Phosgen befreit und in weiteren Destillationsstufen in reines Lösungsmittel, leichtsiedende Nebenprodukte, reines Di- oder Triisocyanat und Schwersieder aufgetrennt. Bevorzugt wird das Isocyanat verwendet.

Die nach dem erfindungsgemäßen Verfahren erhältlichen (cyclo)aliphatischen Isocyanate besitzen Gehalte an Verbindungen mit hydrolysierbarem Chlor von bevorzugt weniger als 200 ppm, besonders bevorzugt weniger als 80 ppm.

Die nach dem erfmdungsgemässen Verfahren erhältlichen aromatischen Isocyanate besitzen Gehalte an Verbindungen mit hydrolysierbarem Chlor von bevorzugt weniger als 100 ppm, besonders bevorzugt weniger als 30 ppm.

Der Gesamtchlorgehalt liegt bei den (cyclo)aliphatischen und bei den aromatischen Isocyanaten bevorzugt unterhalb von 800 ppm, besonders bevorzugt unterhalb von 500 ppm.

Die Bestimmung des Gehalts an hydrolysierbarem Chlor in Isocyanaten im Arbeitsbereich w(Cl) > 5 mg/kg erfolgt durch Urethanisierung, Hydrolyse und potentiometrische Tritration mit Silbernitrat an einer Silber/Silberchlorid-Einstabmesskette.

Zur Bestimmung des Gehalts an hydrolysierbarem Chlor wird die Isocyanatprobe mit Methanol versetzt und 10 min unter Rückfluß urethanisiert. Anschließend wird die Mischung nach Verdünnen mit Wasser durch Kochen unter Rückfluß hydrolysiert. Das hierbei gebildete ionogene Chlor wird nach Ansäuern mit Salpetersäure und Aufstockung mit einer bekannten Masse Natriumchlorid argentometrisch mit einer Silbernitratmasslösung titriert. Die Titration wird mit inkrementeller Reagenzdosierung und automatischer Equivalenzpunkt-Auswertung driftkontrolliert (Gleichgewichtstitration) durchgeführt. Aus der Einwaage der Isocyanatprobe und dem Verbrauch an Silbernitratmasslösung wird der Gehalt an hydrolysierbarem Chlor unter Berücksichtigung der Aufstockung errechnet.

Die Desublimation wird im erfindungsgemäßen Verfahren im sich an die Reaktionszone (K) anschließenden Gasweg durchgeführt. Geeignete Ort sind im Anschluss an die Reaktionszone (K) oder im Anschluss an zwischengeschaltete Apparate wie Kolonnen, Abscheidern oder Pumpen. Beispielhafte Einbauorte sind die in Figur 1 mit (A) bzw. (B) gekennzeichneten Stellen.

Bevorzugt erfolgt die Desublimation hinter dem Isocyanatwäscher (L) oder einem der sich daran anschließenden Apparate im Gasweg.

Die erfindungswesentliche Desublimation der Nebenprodukte und Abtrennung als Festsstoff aus dem Gasstrom wird bevorzugt in einer speziell zu diesem Zweck in das Verfahren eingebrachten Wärmetauscher durchgeführt.

Der Wärmetauscher ist dadurch gekennzeichnet, dass ein Energieübertrag zwischen einem Wärmeträgermedium und dem Prozessstrom stattfindet, wobei Prozessstrom und Wärmeträgermedium voneinander getrennt sind und der Wärmeübergang in der Trennschicht stattfindet.

Geeignet sind Plattenwärmetauscher, die den Prozessstrom an Hohlplatten vorbeileiten, wobei sich das Wärmeträgermedium in den Hohlplatten befindet.

Bevorzugte Plattenwärmetauscher gewährleisten eine vergleichsweise lange Verweilzeit des Prozessgasstromes.

Bevorzugt werden der bzw. die Wärmetauscher daher so ausgelegt und das Verfahren in der Weise betrieben, dass die spezifische Gasgeschwindigkeit 1,5 bis 0,05 m/s, besonders bevorzugt 1,0 bis 0,1 m/s bei Durchgang durch den oder die zur Desublimation eingesetzten Wärmetauscher beträgt.

Bevorzugte Plattenwärmetauscher besitzen eine Oberflächenbeschaffenheit die das Anhaften von Kristallisationskeimen, bevorzugt von Ammoniumhalogeniden, begünstigen. Dies kann beispielsweise durch eine raue Oberfläche erreicht werden, wie sie z.B. durch Strahlvorgänge mit Stoffen wie z.B. Sand oder Granulat erhalten werden.

Bevorzugt besitzen die Plattenwärmetauscher nur ein Minimum an Schweißnähten.

Bevorzugt sind die Plattenwärmetauscher so dimensioniert, dass eine Feststoffabscheidung über einen längeren Zeitraum auch ohne Beeinträchtigung des Strömungsverhaltens des Prozessgases durch den oder die zur Desublimation eingesetzten Wärmetauscher möglich ist.

Bevorzugt besitzen die Wärmetauscher daher ein Plattenregister mit Thermoblechelementen mit einem Verhältnis von Breite zu Länge im Bereich von 0,5 bis 0,05.

Durch den Einsatz von derart dimensionierten Plattenwärmetauschern der vorgenannten Art ist es möglich ohne wesentliche Beeinflussung des Prozessgasstromes über einen Zeitraum von mindestens 3000 h, bevorzugt 3000 bis 6600 h Nebenprodukte durch Desublimation aus dem Gasstrom zu entfernen, wobei dabei wenigstens 60 Gew.-%, bevorzugt wenigstens 80 Gew.-%, besonders bevorzugt wenigstens 95 Gew.-% als Feststoff abscheidbarer Nebenprodukte aus dem Gasstrom entfernt werden.

Bevorzugt werden der oder die zur Desublimation eingesetzten Plattenwärmetauscher als Bypass in den Prozess eingebunden und können so leicht ausgebunden, gereinigt und bei laufendem Prozess wieder eingebunden werden.

Die Reinigung der Platten an der dem Prozessstrom zugewandten Seite erfolgt durch ein Medium, welches die anhaftenden Nebenprodukte ablöst und so eine reinigende Wirkung erzielt wird. Bevorzugt werden die Nebenprodukte dabei ganz oder teilweise aufgelöst und als Lösung oder Slurry aus dem Wärmetauscher ausgeschleust.

Bevorzugt werden dabei zum Auflösen und Ausspülen der Nebenprodukte polare Lösemittel verwendet. Besonders bevorzugt sind polare, protische Lösemittel wie wässrige, basische Lösungen.

Die Geschwindigkeit der Reinigung durch Lösen und Austragen der Nebenprodukte wird durch eine Erhöhung der Fließgeschwindigkeit des Lösemittels beim Reinigungsprozess begünstigt.

Besonders bevorzugt sind Plattenwärmetauscher, die keine oder minimierte Totzonen in der Prozessgas zugewandten Seite haben. Der Einbau kann stehen oder liegend erfolgen.

Neben den durch Desublimation fest sich abscheidenden Nebenprodukten wie Ammoniumhalogeniden können auch kondensierbare Bestandteile in flüssiger Form am Wärmetauscher niedergeschlagen werden. Bevorzugt jedoch wird das erfindungsgemäße Verfahren einschließlich der Prozessparameter der Desublimation eingesetzten Wärmetauscher derart betrieben, dass es an dem oder den zur Desublimation eingesetzten Wärmetauschern nicht zur Absscheidung in flüssiger Form kondensierbarer Bestandteile aus dem Gasstrom kommt. Dies wird bevorzugt derart erreicht, dass der Einbauort des oder der zur Desublimation eingesetzten Wärmetauscher so gewählt wird, dass der Gasstrom frei ist von flüssigen oder in flüssiger Form kondensierbaren Bestandteilen. "Frei von flüssigen oder in flüssiger Form kondensierbaren Bestandteilen" meint, dass der Gasstrom bevorzugt einen Anteil an bei den herrschenden Bedingungen flüssigen oder in flüssiger Form kondensierbaren Bestandteilen von weniger als 15 Gew.- %, besonders bevorzugt weniger als 5 Gew.% aufweist.

Besonders bevorzugt wird daher die Desublimation erst hinter dem Kondensator (M) durchgeführt.

In den erfindungsgemäß zur Desublimation eingesetzten Plattenwärmetauschern können Wärmeträger und Prozessgasstrom im Gegenstromverfahren oder im Gleichstromverfahren geführt werden.

Die Temperatur des Wärmeträgermediums beträgt 100°C bis -40°C, bevorzugt 60°C bis -30°C.

Der Druck des Prozessgases im Wärmetauscher liegt im Bereich von 1500 mbarü bis 200 mbar abs, bevorzugt bei 1000 mbarü bis -500 mbarü, besonders bevorzugt bei 1500 mbar abs bis 700 mbar abs.

Durch den Betrieb eines solchen Plattenwärmetauschers in der oben beschriebenen Art und Weise können in fester Form abscheidbare Nebenprodukte aus dem Prozessgas gezielt abgeschieden werden und dadurch die Verschmutzung nachgeschalteter Anlagenteile, wie Pumpen, Kolonnen, Abscheidern, reduziert werden.

Durch das Betreiben eines Plattenwärmetauschers können so mehr als 60 Gew.-% der abscheidbaren Bestandteile abgeschieden werden. Dieser Anteil kann durch das Betreiben einer Reihenschaltung mehrerer Plattenwärmetauschern auf über 90% gesteigert werden. Das Betreiben in einer Reihenschaltung von 2, 3, 4 oder mehr Wärmetauschern ermöglicht das Ausbinden, Reinigen und Einbinden eines der Wärmetauscher der Reihenschaltung ohne Unterbrechung der Reaktion. Dies kann auch durch eine Parallelschaltung von 2, 3, 4 oder mehr Wärmetauschern erreicht werden, bei der zum Ausbinden, Reinigen und wieder Einbinden eines Wärmetauschers auf den parallel geschalteten Wärmetauscher umgeschaltet wird. Reihen- und Parallelschaltung lassen sich miteinander kombinieren.

Die nach dem erhältlichen Isocyanate lassen sich besonders vorteilhaft bei der Herstellung von Polyurethan lacken sowie Kleb- und Dichtstoffen einsetzen. Dazu werden sie bevorzugt zunächst zu Prepolymeren, Uretdionen, Isocyanuraten, Biureten oder Allophanaten umgesetzt sowie gegebenenfalls mit in der Technik an sich üblichen Methoden blockiert.

### Beispiele:

### Vergleichsbeispiel:

In der Gasphase wurden kontinuierlich 0,6 mol/s Amin (I) und 1,8 mol/s Phosgen (II) bei 300°C Eintrittstemperatur zur Reaktion gebracht, das Reaktionsgas wurde mit Monochlorbenzol (III) niedergeschlagen und die Reaktionsbrüden nach dem in Abbildung 1 dargestellten Fließschema aufgearbeitet. Nach einer Zeit von 25 Tagen wurde ein steigender Differenzdruck im Phosgenwäscher beobachtet, der nach weiteren 6 Tagen ein Abstellen der Reaktion erforderlich machte.

### Beispiel 1:

In der Gasphase wurden kontinuierlich 0,6 mol/s Amin (I) und 1,8 mol/s Phosgen (II) bei 300°C Eintrittstemperatur zur Reaktion gebracht, das Reaktionsgas wurde mit Monochlorbenzol (III) niedergeschlagen und die Reaktionsbrüden nach dem in Abbildung 1 dargestellten Fließschema aufgearbeitet, wobei der erfindungsgemäße Plattenwärmetauscher in Position (A) eingebracht wurde. Er wurde bei Kühlmitteltemperaturen von 40°C und 20°C bei 700mbar abs betrieben. Nach einer Zeit von 31 Tagen konnte ein steigender Differenzdruck im Phosgenwäscher beobachtet werden, der nach weiteren 6 Tagen ein Abstellen der Reaktion erforderte. Eine analytische Auswertung der Ablagerungen in den Apparaten des Abgasweges ergaben, dass 5 Gew.-% bis 20 Gew.-% der insgesamt im Verfahren anfallenden festen Ablagerungen im Plattenwärmetauscher abgeschieden wurden.

### Beispiel 2:

In der Gasphase wurden kontinuierlich 0,6 mol/s Amin (I) und 1,8 mol/s Phosgen (II) bei 300°C Eintrittstemperatur zur Reaktion gebracht, das Reaktionsgas wurde mit Monochlorbenzol (III) niedergeschlagen und die Reaktionsbrüden nach dem in Abbildung 1 dargestellten Fließschema aufgearbeitet, wobei der erfindungswesentliche Plattenwärmetauscher in Position (B) eingebracht wurde. Er wurde bei Kühlmitteltemperaturen von -20°C bei 1200 mbar abs betrieben. Auch nach einer Zeit von 42 Tagen konnte kein Anstieg des Differenzdruckes im Phosgenwäscher beobachtet werden. Eine analytische Auswertung der Ablagerungen in den Apparaten des Abgasweges ergab, dass 60 Gew.-% der gefundenen Ablagerungen im Plattenwärmetauscher abgeschieden wurden.

## Patentansprüche

1. Verfahren zur Herstellung von Isocyanaten durch Phosgenierung von Aminen in der Gasphase, bei dem Nebenprodukte durch Desublimation als Feststoff aus einem oder mehreren Gasströmen abgetrennt werden, worin das zu phosgenicrende Amin und das Phosgen dem Reaktor zugeführt werden, das sich bildende isocyanathaltige Reaktionsgemisch unter Zusatz eines Quenchemittels dem Isocyanatwäscher zugeführt wird und nach Abtrennung des Isocyanats weiter über den Kondensator im Phosgenwäscher in Abgas und Phosgenstrom aufgetrennt wird, **dadurch gekennzeichnet, dass** die Desublimation im sich an die Reaktionszone anschließenden Gasweg in einem oder mehreren Plattenwärmetauschern durchgeführt wird, wobei der bzw. die Wärmetauscher so ausgelegt und das Verfahren in der Weise betrieben wird, dass die spezifische Gasgeschwindigkeit 2 m/s oder weniger bei Durchgang durch den oder die zur Desublimation eingesetzten Wärmetauscher beträgt und die Wärmetauscher mit einem Wärmeträgermedium betrieben werden, das eine Temperatur von 100°C bis - 40°C aufweist.

2. Verfahren gemäß Anspruch 1, **dadurch gekennzeichnet, dass** als Amine Triaminocyclohexan, Tris(aminomethyl)-cyclohexan, Triaminomethylcyclohexan, 1,8-Diamino-4-(aminomethyl)-octan, 1,6,11-Undecantriamin, 1,7-Diamino-4-(3-aminopropyl)heptan. 1,6-Diamino-3-(amino-methyl)-hexan. 1,3,5-Tris(aminomethyl)-cyclohexan, die isomeren Toluylendiamine (TDA), als 2,4-, 2,6-Isomer oder als Gemisch davon, Diaminobenzol, 2,6-Xylidin, Napthylendiamin (NDA), 2,4'- oder 4,4'-Methylen(di-phenylamin) (MDA) oder Isomerengemische davon eingesetzt werden.

3. Verfahren gemäß Anspruch 1 oder 2, **dadurch gekennzeichnet, dass** die Edukte mit einem Inertgas vermischt in die Reaktionszone geführt werden.

4. Verfahren gemäß einem der Ansprüche 1 bis 3, **dadurch gekennzeichnet, dass** die Temperaturen in der Reaktionszone mehr als 260°C aber unterhalb 570°C betragen.

5. Verfahren gemäß einem der Ansprüche 1 bis 4, **dadurch gekennzeichnet, dass** die Desublimation hinter dem Isocyanatwäscher (L) oder einem der sich im Gasweg daran anschließenden Apparate durchgeführt wird.

6. Verfahren gemäß einem der Ansprüche 1 bis 5, **dadurch gekennzeichnet, dass** die Desublimation erst hinter dem Kondensator (M) durchgeführt wird.

7. Verfahren gemäß einem der Ansprüche 1 bis 6, dadurch gekennzeichet, dass es sich bei den abgetrennten Nebenprodukten um Ammonimhalogenide handelt.

## Claims

1. Process for the preparation of isocyanates by phosgenation of amines in the gas phase, in which by-products are separated from one or more gas streams by desublimation as solid, wherein the amine to be phosgenated and the phosgene are fed to the reactor, the resulting isocyanate-containing reaction mixture is fed, with addition of a quenching agent, to the isocyanate scrubber and, after the isocyanate has been separated off, is fed further via the condenser and separated into waste gas and phosgene stream in the phosgene scrubber, **characterized in that** the desublimation is carried out in the gas path after the reaction zone in one or more plate-type heat exchangers, wherein the heat exchanger or heat exchangers is or are designed, and the process is operated, in such a way that the specific gas velocity is 2 m/s or less on passage through the heat exchanger or heat exchangers used for the desublimation and the heat exchangers are operated with a heat transfer medium that has a temperature of 100°C to -40°C.

2. Process according to Claim 1, **characterized in that** triaminocyclohexane, tris(aminomethy1)cyclohexane, triaminomethylcyclohexane, 1,8-diamino-4-(aminomethyl)octane, 1,6,11-undecanetriamine, 1,7-diamino-4-(3-aminopropyl)heptane, 1,6-diamino-3-(aminomethyl)hexane, 1,3,5-tris(aminomethy1)cyclohexane, the isomeric toluenediamines (TDA), as 2,4- or 2,6-isomer or as a mixture thereof, diaminobenzene, 2,6-xylidine, naphthylenediamine (NDA), 2,4'- or 4,4'-methylene(diphenylamine) (MDA) or isomer mixtures thereof are used as amines.

3. Process according to Claim 1 or 2, **characterized in that** the starting materials are fed to the reaction zone as a mixture with an inert gas.

4. Process according to any of Claims 1 to 3, **characterized in that** the temperatures in the reaction zone are greater than 260°C but below 570°C.

5. Process according to any of Claims 1 to 4, **characterized in that** the desublimation is carried out behind the isocyanate scrubber (L) or behind one of the apparatuses thereafter in the gas path.

6. Process according to any of Claims 1 to 5, **characterized in that** the desublimation is carried out only behind the condenser (M).

7. Process according to any of Claims 1 to 6, **characterized in that** the by-products separated off are ammonium halides.

## Revendications

1. Procédé pour la préparation d'isocyanates par phosgénation d'amines en phase gazeuse, dans lequel des produits secondaires sont séparés sous forme solide par désublimation à partir d'un ou de plusieurs flux gazeux, dans lequel l'amine à phosgéner et le phosgène sont introduits dans le réacteur, le mélange réactionnel contenant des isocyanates qui se forme est introduit dans le laveur d'isocyanates avec addition d'un agent de refroidissement brusque et, après la séparation de l'isocyanate, il est séparé davantage via le condenseur dans le laveur de phosgène en effluent gazeux et en flux de phosgène, **caractérisé en ce que** la désublimation est réalisée dans une voie gazeuse consécutive à la zone de réaction, dans un ou plusieurs échangeurs thermiques à plaques, le ou les échangeurs thermiques étant conçus et le procédé étant exploité de manière telle que la vitesse gazeuse spécifique est de 2 m/s ou moins lors du passage au travers du ou des échangeurs thermiques utilisés pour la désublimation et les échangeurs thermiques étant exploités avec un milieu caloporteur qui présente une température de 100°C à -40°C.

2. Procédé selon la revendication 1, **caractérisé en ce qu'**on utilise, comme amines, le triaminocyclohexane, le tris(aminométhyl)-cyclohexane, le triaminométhylcyclohexane, le 1,8-diamino-4-(aminométhyl)-octane, la 1,6,11-undécanetriamine, le 1,7-diamino-4-(3-aminopropyl)heptane, le 1,6-diamino-3-(aminométhyl)-hexane, le 1,3,5-tris(aminométhyl)-cyclohexane, les toluylènediamines isomères (TDA), sous forme d'isomère 2,4, d'isomère 2,6 ou sous forme de mélange de ceux-ci, le diaminobenzène, la 2,6-xylidine, la napthylènediamine (NDA), la 2,4'-méthylène(diphénylamine) ou la 4,4'-méthylène(diphénylamine) (MDA) ou des mélanges d'isomères de celles-ci.

3. Procédé selon la revendication 1 ou 2, **caractérisé en ce que** les produits de départ sont introduits dans la zone de réaction en mélange avec un gaz inerte.

4. Procédé selon l'une quelconque des revendications 1 à 3, **caractérisé en ce que** les températures de la zone de réaction sont supérieures à 260°C mais inférieures à 570°C.

5. Procédé selon l'une quelconque des revendications 1 à 4, **caractérisé en ce que** la désublimation est réalisée en aval du laveur d'isocyanates (L) ou dans un appareil consécutif à celui-ci dans la voie gazeuse.

6. Procédé selon l'une quelconque des revendications 1 à 5, **caractérisé en ce que** la désublimation n'est réalisée qu'en aval du condenseur (M).

7. Procédé selon l'une quelconque des revendications 1 à 6, **caractérisé en ce qu'**il s'agit, pour les produits secondaires séparés, d'halogénures d'ammonium.
